# DEMANDE DE BREVET EUROPEEN

(11) **EP 1 528 133 A1**
(43) Date de publication de la demande: **04.05.2005**
(21) Numéro de dépôt: 04370031.9
(22) Date de dépôt: 29.10.2004
(51) Int. Cl.: D04H 13/00, B32B 5/26, A61F 13/15

(54) **Procédé de fabrication d'un matériau complexe obtenu par lamination d'un film élastique pris en sandwich entre deux nappes de non-tissé**

(30) Priorité: 29.10.2003 FR 0312662
(71) Demandeur: Chabe Conception Process Ingénierie et Développement (Chabe CPID), Société par Actions Simplifiée, 62820 Libercourt (FR)
(72) Inventeur: Daada, Nede, 59000 Lille (FR); Chabierski, Pascal, 62590 Oignies (FR)
(74) Mandataire: Duthoit, Michel

(57) **Abrégé**

La présente invention concerne un procédé de fabrication d'un matériau complexe multicouche, au moins tri-couche, obtenu par lamination d'un film élastique pris en sandwich entre deux nappes de non-tissé.

Selon l'invention, on soumet les deux nappes de non-tissé qui ne présentent pas une mémoire de forme mais qui présentent un aspect textile et le film élastique, qui présente une mémoire de forme, mais qui ne présente pas d'aspect textile, de laizes identiques à un calandrage afin de les perforer et simultanément les lier ponctuellement.

## Description

La présente invention concerne un procédé de fabrication d'un matériau complexe multicouche, au moins tri-couche, à savoir un film élastique pris en sandwich entre deux nappes de non-tissé, et un matériau complexe ainsi obtenu.

L'invention concerne également une machine conçue pour la fabrication dudit matériau complexe.

L'invention concerne, par ailleurs, une application dudit matériau complexe pour la fabrication des couches-culottes ou de sous-vêtements jetables, ainsi que leur procédé de fabrication.

Dans le domaine de la fabrication des sous-vêtements jetables, il est connu d'utiliser un matériau non-tissé non extensible bordé d'élastiques, comme le serait un tel sous-vêtement textile.

Actuellement, dans le domaine de la fabrication de couches-culottes, il est connu d'utiliser un matériau complexe élastique formé de deux nappe de non-tissé et un film intermédiaire élastique.

Pour ce, il est connu l'assemblage des différentes couches d'un complexe laminé par collage, calandrage thermique ou soudure ultrasonique, et la présence d'une multiplicité de sites discrets de liaison thermique distribués dans la totalité du complexe.

Bien que connu, le procédé de fabrication d'un tel matériau complexe, ainsi que la fabrication de ces sous-vêtements à usage unique et des couches-culottes, présentent plusieurs inconvénients.

Tout d'abord, la perforation du matériau complexe et son assemblage sont réalisés en deux étapes différentes, voire avec apport d'agent de collage, ce qui constitue une nette diminution dans le rendement et dans la productivité de ladite fabrication, augmente le coût de revient et altère les caractéristiques du produit final.

En outre, l'assemblage des trois couches par calandrage thermique est réalisé à une température différente de la température de fusion de la couche intermédiaire élastique, ce qui rend le processus plus complexe et moins efficace.

Par ailleurs, dans les complexes non-tissés, l'utilisation des fibres de polyester est généralement connue. Toutefois, la présence d'un film polyester-viscose n'est pas suggérée. Or, les inventeurs ont remarqué que l'absence de viscose dans la nappe de non-tissé, qui constitue le matériau complexe, présente de nombreux inconvénients, notamment au niveau de la lamination et de l'allongement dudit matériau complexe.

De plus, les sous-vêtements à usage unique et les couches-culottes fabriqués à partir d'un matériau complexe obtenu par collage présentent plusieurs inconvénients, tels que l'effilochage des bords et les surépaisseurs dues à la couture, ainsi qu'une certaine rigidité.

Le but de l'invention est de proposer un procédé de fabrication d'un matériau complexe multicouche, au moins tri-couche, utilisé dans la fabrication des sous-vêtements à usage unique ou des couches-culottes, qui sont de plus en plus demandés, car ils sont faciles à utiliser et sont très économiques, et qui permettent simultanément de pallier tous les inconvénients mentionnés ci-dessus, notamment au niveau de la productivité et de l'allongement de matériaux complexes.

Un autre but de la présente invention est de proposer l'utilisation d'un non-tissé obtenu, par voie humide ou non, à partir de fibres mélangées d'origine chimique, synthétique, tel que le polyéthylène, polypropylène ou tout autre matériau polymère, ou naturelle, avantageusement à partir d'un mélange de viscose et de polyester dans la fabrication dudit matériau complexe, afin d'autoriser un aspect textile à l'ensemble. En outre, le non-tissé présente une structure pleine ou ouverte, avantageusement ouverte, ce qui permet de favoriser sa déformation et son élasticité dans le sens de travers, c'est-à-dire, dans le sens de la largeur du sous-vêtement ou de la couche-culotte.

La mise en oeuvre de la présente invention permet d'obtenir des couches-culottes ou des sous-vêtements élastiques sans le besoin d'ajouter une bande élastique autour de la ceinture dans le cas des couches-culottes, et de la ceinture et de l'entrejambe dans le cas des sous-vêtements.

Un autre but de l'invention est de proposer une machine capable de fabriquer un tel matériau complexe, notamment une machine capable d'effectuer la liaison des différentes couches du matériau et la perforation dudit matériau simultanément, tout en préservant une bonne élasticité.

Un autre but de l'invention est de proposer un procédé de fabrication de couches-culottes ou de sous-vêtements jetables à partir dudit matériau complexe.

Un avantage de la présente invention réside dans la flexibilité du procédé de fabrication qui permet d'obtenir toutes sortes de modèles de culottes sans aucune limitation.

D'autres buts et avantages de l'invention apparaîtront au cours de la description qui va suivre, qui n'est donnée qu'à titre indicatif et qui n'a pas pour but de la limiter.

L'invention concerne un procédé de fabrication d'un matériau complexe multicouche, au moins tri-couche, obtenu par lamination d'un film élastique pris en sandwich entre deux nappes de non-tissé, caractérisé par le fait que l'on soumet les deux nappes de non-tissé, qui ne présentent pas une mémoire de forme mais qui présentent un aspect textile, et le film élastique, qui présente une mémoire de forme mais qui ne présente pas d'aspect textile, de laizes identiques à un calandrage afin de les perforer et simultanément les lier ponctuellement, pour aboutir à un matériau complexe avec mémoire de forme et toucher textile.

L'invention concerne également un matériau complexe, caractérisé par le fait que les deux nappes de non-tissé sont obtenues, par voie humide ou non, à partir de fibres mélangées d'origine chimique, synthétique ou naturelle, avantageusement à partir d'un mélange de viscose et de polyester.

L'invention concerne par ailleurs une machine conçue pour la fabrication dudit matériau complexe, caractérisée par le fait qu'elle comprend :
- des moyens d'alimentation de deux nappes de non-tissé et d'un film élastique,
- des moyens pour superposer les deux nappes de non-tissé et le film élastique,
- des moyens pour soumettre les deux nappes de non-tissé et le film élastique à un calandrage afin de les perforer et simultanément les lier ponctuellement.

L'invention concerne en outre l'application dudit matériau complexe à la fabrication de couches-culottes ou de sous-vêtements.

L'invention concerne encore un procédé de fabrication de sous-vêtements jetables, caractérisé par le fait que :
- les sous-vêtements sont fabriqués selon un processus en continu en déroulant tout d'abord les deux non-tissés ainsi que le film élastique pris en sandwich entre ceux-ci,
- on applique un non-tissé en coton sur la surface supérieure du complexe, notamment pour renforcer la partie qui constituera le centre de la culotte, enduit préalablement de colle,
- on effectue la découpe du sous-vêtement, puis la séparation de chaque produit par découpe de la bande,
- on rabat l'arrière sur l'avant pour former les sous-vêtements et on assemble les bords.

L'invention concerne également un procédé de fabrication de couches-culottes comprenant une bande centrale, deux panneaux élastiques et deux « oreilles » afin de former la ceinture, caractérisé par le fait que :
- les deux « oreilles » sont fabriquées à partir dudit matériau complexe,
- on utilise deux rubans crochets attachés aux deux panneaux élastiques afin de serrer les couches-culottes autour de la ceinture.

L'invention sera mieux comprise à la lecture de la description suivante accompagnée de dessins en annexe parmi lesquels :
- la figure 1 illustre de manière schématique la structure du non-tissé,
- la figure 2 illustre de manière schématique une machine conçue pour la fabrication du matériau complexe par calandrage thermique de la présente invention,
- la figure 3 illustre de manière schématique une machine conçue pour la fabrication du matériau complexe par calandrage ultrasonique de la présente invention,
- les figures 4a et 4b illustrent deux variétés de motifs utilisés sur la surface d'un des cylindres lors du calandrage,
- la figure 5 illustre de manière schématique un sous-vêtement, tel qu'une culotte, à plat,
- la figure 6 illustre de manière schématique un sous-vêtement, tel qu'une culotte à plat avec renfort,
- la figure 7 illustre de manière schématique un sous-vêtement fini, tel qu'illustré aux figures 5 et 6, montrant les zones d'assemblage,
- la figure 8 illustre de manière schématique une couche-culotte pour bébé.

L'invention concerne tout d'abord un procédé de fabrication d'un matériau complexe élastique multicouche, au moins tri-couche, obtenu par lamination d'un film élastique pris en sandwich entre deux nappes de non-tissé.

Plus précisément, on superpose deux nappes de non-tissé, qui ne présentent pas une mémoire de forme mais qui présentent un aspect textile, entre lesquelles on dispose le film élastique, qui présente une mémoire de forme, mais qui ne présente pas d'aspect textile, les deux nappes et le film étant mis au contact l'un de l'autre. Ensuite, selon l'invention, on soumet les deux nappes de non-tissé et le film élastique à un calandrage afin de les perforer et simultanément les lier ponctuellement, pour aboutir à un matériau complexe.

On a obtenu de bons résultats avec une largeur du complexe allant jusqu'à 420 mm.

Pour pouvoir fabriquer un non-tissé élastique, il est nécessaire de disposer d'un non-tissé présentant des caractéristiques physiques, chimiques et mécaniques particulières.

Selon l'invention, les matières primaires utilisées dans la fabrication du matériau complexe multicouche ne sont ni tendues, ni grattées, ni extrudées. Dans le cas du non-tissé, les matières premières sont consolidées en amont de la phase de laminage. Les différentes matières premières sont dans leur état de repos sans déformation en amont.

Selon l'invention, le matériau complexe est notamment destiné à être utilisé dans la fabrication des couches-culottes ou des sous-vêtements, De ce fait, on a choisi avantageusement des fibres donnant des caractéristiques de toucher et d'aspect textile avec un faible coût de revient. Afin d'obtenir ces caractéristiques, les non-tissés pourront être obtenus par voie humide.

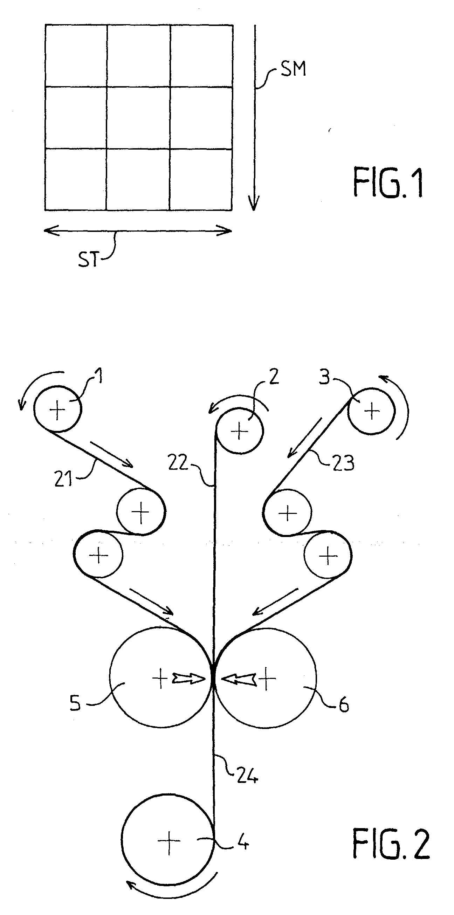

L'intérêt d'un système par voie humide est d'obtenir une étoffe flexible, résistante, ayant un toucher textile avec une déformation en sens travers ST supérieure à 50 %, alors qu'elle n'autorise quasiment pas d'extension dans le sens machine SM. De plus, l'utilisation d'un non-tissé ayant une structure pleine ou ouverte, avantageusement ouverte, permet d'augmenter la déformation en sens travers ST jusqu'à 100 %, comme illustré à la figure 1.

Selon l'invention, les fibres utilisées pour la fabrication de non-tissés peuvent se présenter sous la forme d'un mélange de fibres d'origine chimique, synthétique ou naturelle, avantageusement sous la forme d'un mélange de viscose et de polyester.

On a obtenu de bons résultats lorsque la viscose et le polyester sont présents dans les limites pondérales suivantes :
- viscose : 10 à 50 %, préférentiellement 30 %,
- polyester : 50 à 90 %, préférentiellement 70 %.

La viscose est une fibre chimique composée de cellulose régénérée. Pour fabriquer cette fibre, on commence par fabriquer de l'alcali-cellulose en traitant par soude caustique sous agitation mécanique de linters de coton. Cette pâte est ensuite passée dans une filière et étirée.

Par linters, on entend des fibres très courtes qui ne sont pas détachées des graines de coton durant le premier égrenage.

Malgré l'étirage, les fibres de viscose sont moins orientées que les fibres de coton et, selon l'invention, cela présente l'avantage de présenter plus de zones amorphes et par conséquent de faciliter la lamination.

Un des avantages est le fait que la fibre de viscose présente une aptitude à l'allongement de 12 à 25 %.

Un autre avantage réside dans le fait que la viscose n'a pas de point de fusion. Cette caractéristique permet de lier les différentes couches sans altérer la viscose, et surtout sans avoir de problème avec cette fibre lors de liages thermiques.

D'autres avantages résident dans le fait que la viscose présente une bonne résistance à l'usure par abrasion et à la lumière, un entretien facile et une grande facilité de teinture.

Selon la présente invention, le polyester utilisé dans la fabrication de matériaux complexes est d'origine chimique, et est notamment fabriqué par polycondensation de deux monomères, à savoir: l'acide téréphtalique et l'éthylène glycol.

Contrairement à la fabrication de la viscose, les filaments de polyester sont filés par fusion, puis étirés à plusieurs reprises afin d'augmenter plusieurs fois leur longueur par rapport à leur longueur initiale, ce qui oriente les molécules à chaîne longue et confère une résistance aux fibres.

Les fibres de polyester présentent une haute résistance et sont également résistantes au rétrécissement et à l'allongement. Les tissus sèchent rapidement et présentent une tendance à l'auto-défroissabilité et à la rétention du pli.

Le tableau suivant montre les caractéristiques principales de polyester.

**TABLEAU**

| | |
|---|---|
| Absorption d'eau faible : | Taux de reprise 3 % |
| Excellente thermoplasticité | Possibilité de thermofixage, de plissage permanent |
| Température transition vitreuse Tg : | environ 80°C |
| Point de fusion | environ 260°C |
| Ramollissement | environ 230°C |
| Dégradation : | environ 300°C |
| Inflammabilité | Difficilement inflammable (sauf en mélange avec des fibres cellulosiques) Formation de boules dures de matière fondue |
| Infroissabilité | Très bonne |
| Stabilité dimensionnelle | Très bonne |
| Fluage | Faible |
| Allongement à la rupture | 14 à 30 % (7 à 15 % pour les fils haute ténacité) Variation en fonction du procédé de stabilisation-relaxation appliqué au fil par traitement thermique |

Selon la présente invention, afin de lier les différentes nappes pour obtenir le matériau complexe, on utilise soit un calandrage thermique, soit un calandrage par ondes hertziennes et/ou électromagnétiques et/ou le collage.

En particulier, selon l'invention, le calandrage thermique permet de lier les trois nappes qui constituent le matériau complexe par le biais du film élastique pris en sandwich entre les deux nappes de non-tissé.

Le calandrage thermique s'effectue à une température proche de la température de fusion du film élastique (130-133 °C), ce qui permet de le transformer en thermoliant.

Parmi les ondes hertziennes et/ou électromagnétiques utilisées pour le calandrage, les ultrasons sont particulièrement utilisés dans le domaine industriel, notamment dans l'industrie textile, dus à l'échauffement ou les vibrations qu'ils induisent.

Selon la présente invention, l'utilisation d'un calandrage par ultrasons a la finalité de souder ponctuellement les différentes couches qui constituent le matériau complexe mais aussi de les perforer simultanément.

Les points de soudure ou de liaison peuvent être circulaires, carrées, triangulaires, ou présenter toute autre forme géométrique et peuvent être distribués aléatoirement. Toutefois, le pourcentage de points de liaison ne doit pas dépasser 10 % de la surface totale du matériau.

Par ailleurs, l'utilisation d'un calandrage par ultrasons a l'avantage d'être le plus adapté pour assembler des pièces thermofusibles.

Selon la présente invention, seul le polyester est thermofusible. De ce fait, la soudure lie uniquement les fibres de polyester entre elles. Les fibres de viscose ne contribuent donc pas à la liaison du matériau complexe.

Le film élastique pris en sandwich entre les deux nappes de non-tissé est inerte aux ondes ultrasons et n'a pas la capacité de réfléchir les ondes ultrasons. Le film élastique laisse donc traverser les ondes mais lorsque les ondes traversent ce dernier, elles perforent le film élastique.

En utilisant ce type de matériau, on effectue donc en une seule étape le soudage et la perforation, au lieu de deux étapes si l'on avait pris d'autres natures de fibres ou un autre type de film élastique.

La perforation du matériau complexe permet la respirabilité du produit.

En outre, selon la présente invention, le procédé de calandrage par ultrasons permet une rapidité d'exécution et donne une soudure propre, le temps de soudage par points étant inférieur à la seconde.

Enfin, le soudage est continu grâce à un outil ultrason rotatif. Sur les matériaux choisis, on peut donc faire varier la vitesse de soudage notamment de quelques mètres à plus de 200 m/mm.

Le principe de calandrage par ultrasons consiste à associer une pression d'appui à la vibration ultrasonore. L'énergie ultrasons, compte tenu des caractéristiques des matériaux, produit un échauffement intense dans la matière. Elle fusionne à l'interface de l'assemblage et donc le soudage par ultrasons ne nécessite pas d'agent de liaison complémentaire. La force de pression est produite manuellement ou par un vérin.

Un autre but de la présente invention est de proposer une machine capable de fabriquer un tel matériau complexe, par calandrage thermique ou calandrage ultrasonique.

Comme illustrée à la figure 2, ladite machine conçue pour la fabrication du matériau complexe 24 par calandrage thermique est notamment composée de quatre rouleaux principaux 1, 2, 3 et 4 et de deux cylindres 5 et 6. Les quatre rouleaux principaux sont constitués respectivement par une bobine 1 d'une nappe de non-tissé 21, une bobine 2 de film élastique 22, une bobine 3 d'une deuxième nappe de non-tissé 23 et une bobine 4 dudit matériau 24 complexe obtenu après le calandrage.

Les deux cylindres 5 et 6 ont comme fonction d'appliquer une pression sur les deux nappes 21, 23 de non-tissé et le film élastique 22, pris en sandwich entre elles, les uns contre les autres, de les calandrer et perforer simultanément.

Selon la présente invention, le calandrage peut être thermique ou par ultrasons. Le calandrage thermique est particulièrement adapté à la variante de réalisation où le non-tissé comprend uniquement du polypropylène. Le calandrage par ultrasons est plus adapté à une variante de réalisation où le non-tissé comprend un mélange de polypropylène et de polyester. Toutefois, les deux techniques peuvent s'utiliser dans les deux cas.

Selon la présente invention, la machine conçue pour la fabrication dudit matériau complexe avec un calandrage thermique, telle qu'illustrée à la figure 2, présente des moyens d'alimentation de deux nappes de non-tissé 21, 23 et d'un film élastique 22, substantiellement constitués desdites bobines précitées 1, 2, 3, des moyens pour superposer deux nappes de non-tissé et le film élastique, et des moyens pour soumettre les deux nappes de non-tissé et le film élastique à un calandrage afin de les perforer et simultanément les lier ponctuellement.

Lesdits moyens pour presser et chauffer l'ensemble des nappes et le film élastique à une température voisine de la température de fusion dudit film élastique se présentent sous la forme de deux cylindres chauffants 5, 6, tels qu'illustrés à la figure 2, appliqués l'un vers l'autre, assujettis à des moyens pour contrôler la pression entre lesdits deux cylindres.

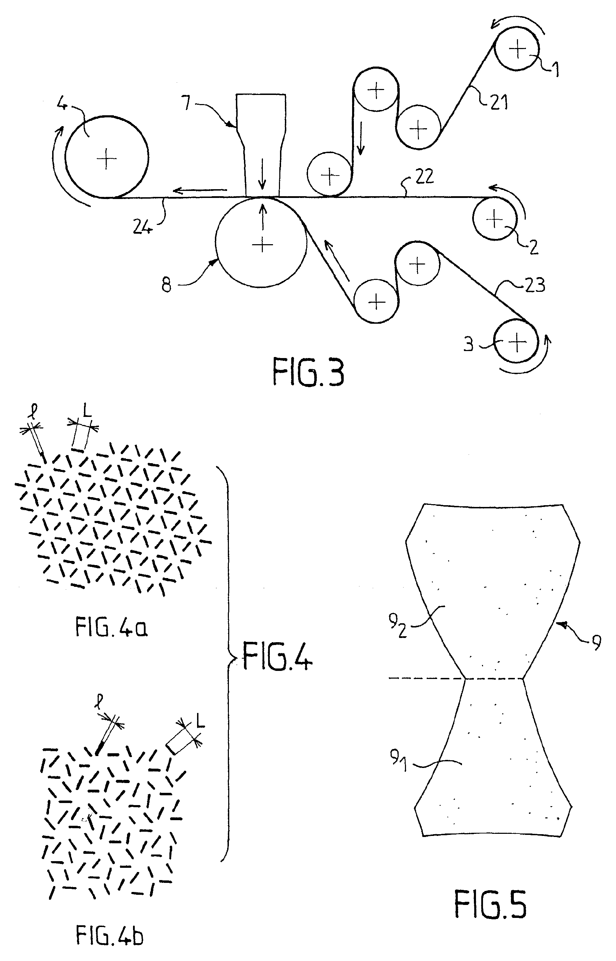

Cela étant, selon une autre variante de la présente invention, la machine conçue pour la fabrication dudit matériau complexe, telle qu'illustrée à la figure 3, permet de transmettre des vibrations mécaniques à une fréquence ultrasonore de l'ordre de 20.000 hertz par le biais d'une sonotrode 7, et de moyens de réflexion 8 des vibrations mécaniques de fréquence ultrasonore.

Elle comporte en outre des moyens permettant de maintenir un écartement constant entre les moyens permettant de transmettre les vibrations mécaniques ultrasonores et les moyens de réflexion afin d'éviter le contact direct, et des moyens pour appliquer les deux nappes et le film élastique sur lesdits moyens de réflexion.

Par vibration mécanique à une fréquence ultrasonore, on entend des vibrations mécaniques semblables à celles produites par n'importe quel instrument de musique, mais parce qu'elles sont à une fréquence supérieure aux capacités audibles de l'oreille humaine (supérieure à 16 kh), elles sont appelées ultrasons. Ces ondes sont produites par un générateur. Ce dernier envoie un courant alterné ayant la même fréquence que le convertisseur qui les transforme en vibrations mécaniques. Une fois la vibration produite, elle est ensuite amplifiée et transmise aux pièces à souder par la sonotrode 7.

La technique de soudure par ultrasons est faite de plusieurs étapes :
- les deux nappes 21, 23 de non-tissé et le film élastique 22 à souder sont placés sous la sonotrode 7 et le cylindre 8 qui sert aussi de moyens de réflexion,
- la sonotrode 7 descend et une pression est appliquée sur les nappes à souder,
- la sonotrode 7 applique les ultrasons auxdites nappes,
- la pression est maintenue pendant la période de refroidissement,
- la sonotrode 7 remonte.

La liaison est obtenue en quelques dixièmes de secondes, résiste à l'arrachement et est étanche.

En outre, selon une caractéristique de l'invention, l'un des cylindres 5, 6, tels qu'illustrés à la figure 2, ou le cylindre 8, tel qu'illustré à la figure 3, présente une surface externe à picots avec une répartition périodique, selon un motif préétabli, tel qu'illustré aux figures 4a ou 4b, afin de permettre la perforation du matériau complexe obtenu par lamination au moment du soudage thermique ou ultrasonique et afin de conférer plus d'élasticité au matériau laminé. C'est ce motif préétabli que l'on retrouvera sur ledit complexe.

Il s'agit par exemple de segments, de l'ordre de 1 à 3 mm de longueur et de 0,2 à 0,6 mm de largeur, disposés radialement ou en rosace selon une densité plus ou moins importante, de l'ordre de 5 à 10 segments par cm², selon l'élasticité souhaitée.

Un autre but de l'invention est d'appliquer le matériau complexe obtenu par soudage thermique ou par ultrasons à la fabrication des couches-culottes ou de sous-vêtements.

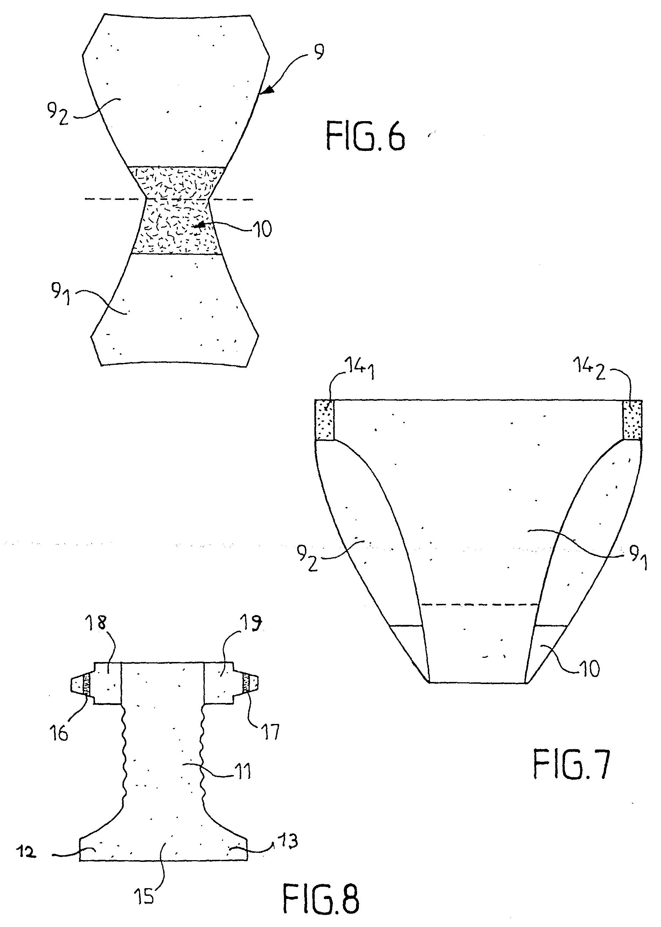

En particulier, selon la présente invention, le procédé de fabrication concerne notamment les sous-vêtements jetables, par exemple des culottes, telles qu'illustrées à plat à la figure 5, mais ce qui n'est pas limitatif, qui présente une zone 9₁ et une zone arrière 9₂.

Plus précisément, selon l'invention, les culottes obtenues par le présent procédé peuvent comporter deux pièces, telles qu'illustrées à plat à la figure 6, une pièce destinée à former le slip 9 et une pièce de renfort en coton 10.

Selon la présente invention, la fabrication de sous-vêtements est réalisée selon un processus en continu qui comporte notamment la succession des étapes suivantes.

Les deux nappes de non-tissé ainsi que le film élastique pris en sandwich sont laminés afin d'obtenir le matériau complexe tel que décrit ci-dessus.

Une fois le matériau complexe obtenu, on applique un renfort de non-tissé en coton 10 sur la surface supérieure du matériau complexe. Afin de maintenir le renfort sur le complexe, on l'enduit préalablement de colle. Le coton de renfort 10 étant une fibre naturelle, est notamment utilisé dans ce cas pour des raisons d'hygiène.

On effectue alors la découpe de sous-vêtements, notamment la coupe de l'entrejambe, suivie de la séparation de chaque produit par découpe de la bande.

Finalement, on rabat l'arrière 9₁ de la pièce découpée sur l'avant 9₂ bord à bord de façon à ce que les lisières coïncident, tel qu'illustré à la figure 7, et ensuite on assemble les bords par ultrasons.

La forme de la pièce 9 est obtenue grâce à une coupe et une découpe, effectuée dans la laize. Cette pièce 9 est coupée de façon à former un slip traditionnel. Cette forme de slip est obtenue grâce à une coupe par ultrasons ou au couteau rotatif. Ce type de coupe évite au non-tissé de s'effilocher.

De plus, le matériau utilisé étant laminé tel que décrit ci-dessus avec une matière élastique afin de rendre l'ensemble élastique, la culotte ne nécessite pas d'ajout d'élastiques au niveau de la taille ou de l'entrejambe, comme il est d'usage de le voir sur les sous-vêtements réutilisables connus.

Ainsi coupée, on vient rabattre la partie arrière 9₂ sur la partie avant 9₁ et on effectue la soudure au niveau de la ceinture. Ces liaisons, repérées 14₁ et 14₂, sont faites par ultrasons sur une largeur de 8 à 10 mm, tel qu'illustré à la figure 7.

Un avantage de ce type de liaison par ultrasons par rapport à une liaison traditionnelle par collage réside dans le fait que c'est que la liaison par ultrasons évite les surépaisseurs car cette technique permet de compacter en même temps que la soudure s'effectue.

Ces sous-vêtements restent quasi invisibles lors du port de vêtements collants.

Les figures 5, 6 et 7 représentent, à titre d'exemple, une forme d'exécution de la culotte, mais d'autres modes de mise en oeuvre, à la portée de l'homme de l'art, auraient pu être envisagés sans pour autant sortir du cadre de l'invention.

Cela étant, le but de l'invention est aussi de proposer un procédé de fabrication de couches-culottes qui comprennent un élément central 11, deux panneaux élastiques 18,19 et éventuellement deux « oreilles » 12 et 13 afin de former la ceinture, tel qu'illustré à la figure 8.

L'élément central 11 est formé, selon une structure connue, d'un film en polyéthylène extérieur imperméable, d'un tampon absorbant et d'un voile non-tissé interne perméable au liquide.

La ceinture de la couche-culotte est formée par l'élément central 11, les deux panneaux élastiques 18, 19 et éventuellement les deux « oreilles » 12 et 13. Les panneaux élastiques 18, 19 sont avantageusement fabriqués à partir d'un matériau complexe décrit ci-dessus, disposés transversalement et vers l'extérieur dudit élément central 11, et vont coopérer avec la partie avant 15 de la couche-culotte, et éventuellement les deux oreilles 12, 13.

Le scellage des bords de la culotte et la fixation des panneaux élastiques 18, 19 sur la couche se font par scellage à froid, pression à chaud, soudure ultrasons ou collage hotmelt.

Le système de fermeture est constitué de deux attaches 16, 17 fixées sur les panneaux élastiques 18, 19 coopérant avec au moins une zone à boucles, fixée sur le film de polyéthylène extérieur de la couche-culotte, selon un principe de fermeture connu sous le nom « Velcro ».

Naturellement, d'autres modes de mise en oeuvre, à la portée de l'homme de l'art, auraient pu être envisagés sans pour autant sortir du cadre de l'invention.

## Revendications

1. Procédé de fabrication d'un matériau complexe multicouche, au moins tri-couche, obtenu par lamination d'un film élastique (22) pris en sandwich entre deux nappes de non-tissé (21, 23), **caractérisé par le fait que** l'on soumet les deux nappes de non-tissé (21, 23), qui ne présentent pas une mémoire de forme mais qui présentent un aspect textile, et le film élastique (22), qui présente une mémoire de forme, mais qui ne présente pas d'aspect textile, de laizes identiques à un calandrage afin de les perforer et simultanément les lier ponctuellement, pour aboutir à un matériau complexe avec mémoire de forme et toucher textile.

2. Procédé de fabrication d'un matériau complexe selon la revendication 1, **caractérisé par le fait que** l'on soumet les deux nappes de non-tissé (21, 23) et le film élastique (22) à un calandrage thermique.

3. Procédé de fabrication d'un matériau complexe selon la revendication 1, **caractérisé par le fait que** l'on soumet les deux nappes de non-tissé (21, 23) et le film élastique (22) à un calandrage par ondes hertziennes et/ou électromagnétiques.

4. Procédé de fabrication d'un matériau complexe selon la revendication 2, **caractérisé par le fait que** l'on effectue le calandrage thermique à une température voisine de la température de fusion dudit film élastique (22).

5. Procédé de fabrication d'un matériau complexe selon la revendication 3, **caractérisé par le fait que** l'on soumet les deux nappes de non-tissé (21, 23) et le film élastique (22) à un calandrage par ultrasons.

6. Matériau complexe obtenu selon l'une quelconque des revendications précédentes, **caractérisé par le fait que** les deux nappes de non-tissé (21, 23) sont obtenues par voie humide ou non à partir de fibres mélangées d'origine chimique, synthétique ou naturelle, avantageusement à partir d'un mélange de viscose et de polyester.

7. Matériau complexe selon la revendication 6, **caractérisé par le fait que** la viscose et le polyester sont présents dans les limites pondérales indiquées :
- viscose : 10 à 50 %, préférentiellement 30 %
- polyester : 50 à 90 %, préférentiellement 70 %.

8. Machine pour la fabrication dudit matériau complexe obtenu selon la revendication 2, **caractérisée par le fait qu'**elle comprend :
- des moyens d'alimentation de deux nappes de non-tissé (21, 23) et d'un film élastique (22),
- des moyens pour superposer deux nappes de non-tissé (21, 23) et le film élastique,
- des moyens pour soumettre les deux nappes de non-tissé (21, 23) et le film élastique (22) à un calandrage afin de les perforer et simultanément les lier ponctuellement.

9. Machine selon la revendication 8, **caractérisée par le fait qu'**elle comprend :
- des moyens pour chauffer l'ensemble des nappes et le film élastique (22) à une température voisine de la température de fusion dudit film élastique (22),
- des moyens pour presser les deux nappes (21, 23) et le film élastique (22).

10. Machine selon la revendication 9, **caractérisée par le fait que** lesdits moyens pour chauffer et lesdits moyens pour presser se présentent sous la forme de deux cylindres chauffants 5, 6) appliqués l'un vers l'autre, assujettis à des moyens pour contrôler la pression entre lesdits deux cylindres.

11. Machine pour la fabrication dudit matériau complexe selon la revendication 8, **caractérisée par le fait qu'**elle comprend :
- des moyens permettant de transmettre des vibrations mécaniques à une fréquence ultrasonore, de l'ordre de 20.000 hertz,
- des moyens de réflexion (8) des vibrations mécaniques de fréquence ultrasonore,
- des moyens permettant de maintenir un écartement constant entre les moyens permettant de transmettre les vibrations mécaniques ultrasonores et les moyens de réflexion afin d'éviter le contact direct,
- des moyens pour appliquer les deux nappes (21, 23) et le film élastique (22), sur lesdits moyens de réflexion (8).

12. Machine pour la fabrication dudit matériau complexe selon la revendication 11, **caractérisée par le fait que** :
- les moyens permettant de transmettre les vibrations mécaniques à une fréquence ultrasonore se présentent sous la forme de sonotrodes (7),
- les moyens de réflexion se présentent sous la forme d'un cylindre (8), sur lequel sont appliqués les deux nappes (21, 23) et le film élastique (22).

13. Machine selon la revendication 10 ou 12, **caractérisée par le fait qu'**un des cylindres (5, 6, 8) présente une surface externe à picots, avec une répartition périodique, selon un motif préétabli (4a, 4b) afin de permettre la perforation du matériau complexe obtenu par lamination au moment du soudage thermique ou ultrasonique et afin de conférer plus d'élasticité au matériau laminé.

14. Application du matériau complexe obtenu selon la revendication 1 à la fabrication de couches-culottes, ou de sous-vêtements.

15. Application du matériau complexe obtenu selon la revendication 2 à la fabrication de couches-culottes.

16. Application du matériau complexe selon la revendication 3 à la fabrication de sous-vêtements.

17. Procédé de fabrication des sous-vêtements jetables selon la revendication 14 ou 16, **caractérisé par le fait que** :
- les sous-vêtements sont fabriqués selon un processus en continu en déroulant tout d'abord les deux non-tissés (21, 23) ainsi que le film élastique (22) pris en sandwich entre ceux-ci,
- on applique un non-tissé en coton (10) sur la surface supérieure du complexe, notamment pour renforcer la partie qui constituera le centre de la culotte, enduit préalablement de colle,
- on effectue la découpe du sous-vêtement, puis la séparation de chaque produit par découpe de la bande,
- on rabat l'arrière (9₂) sur l'avant (9₁) pour former les sous-vêtements et on assemble les bords par soudure au niveau de la ceinture (14₁, 14₂).

18. Procédé selon la revendication 17, **caractérisé par le fait que** l'assemblage des sous-vêtements est effectué par ultrasons.

19. Procédé de fabrication de couches-culottes selon la revendication 14 ou 15 comprenant une bande centrale (11) et deux oreilles (12, 13) afin de former la ceinture, **caractérisé par le fait que** :
- les deux oreilles (12, 13) sont fabriquées à partir dudit matériau complexe,
- on utilise deux attaches (16, 17) à crochets fixées sur les oreilles (12, 13) coopérant avec une zone à boucles afin de serrer les couches-culottes autour de la ceinture.
